# FASCICULE DE BREVET EUROPEEN

(11) **EP 2 791 356 B1**
(45) Date de publication et mention de la délivrance du brevet: **29.11.2017**
(21) Numéro de dépôt: 12815741.9
(22) Date de dépôt: 14.12.2012
(51) Int. Cl.: C12Q 1/68

(54) **PROCEDE D'AMPLIFICATION TRANSCRIPTIONNELLE D'ACIDES NUCLEIQUES REUNISSANT DES ETAPES DE TEMPERATURES DIFFERENTES**
VERFAHREN ZUR TRANSKRIPTIONELLEN AMPLIFIKATION VON NUKLEINSÄUREN MITTELS KOMBINATION VERSCHIEDENER TEMPERATURSCHRITTE
METHOD FOR TRANSCRIPTIONAL AMPLIFICATION OF NUCLEIC ACIDS COMBINING DIFFERENT TEMPERATURE STEPS

(30) Priorité: 16.12.2011 FR 1161758
(43) Date de publication de la demande: 22.10.2014
(73) Titulaire: Biomérieux, 69280 Marcy l'Étoile (FR)
(72) Inventeur: LAAYOUN, Ali, F-38260 La Frette (FR); LAURENT, Alain, 38100 Grenoble (FR); MESTA, Laurent, F-69740 Genas (FR)
(86) Numéro de dépôt international: PCT/FR2012/052934
(87) Numéro de publication internationale: WO 2013/088085

(56) Documents cités:
- EP-A2- 0 821 059
- WO-A1-00/36112
- US-A1- 2002 132 242
- LANGABEER: "Transcription-mediated amplification and hybridisation protection assay to determine BCR-ABL transcript levels in patients with chronic myeloid leukaemia", LEUKEMIA, 1 janvier 2002 (2002-01-01), pages 393-399, XP055035200, DOI: 10.1038/sj/leu/2402392
- CARNINCI P ET AL: "THERMOSTABILIZATION AND THERMOACTIVATION OF THERMOLABILE ENZYMES BY TREHALOSE AND ITS APPLICATION FOR THE SYNTHESIS OF FULL LENGTH CDNA", PROCEEDINGS OF THE NATIONAL ACADEMY OF SCIENCES, NATIONAL ACADEMY OF SCIENCES, US, vol. 95, 1 janvier 1998 (1998-01-01), pages 520-524, XP002941376, ISSN: 0027-8424, DOI: 10.1073/PNAS.95.2.520
- A SPIESS ET AL: "A Highly Efficient Method for Long-Chain cDNA Synthesis Using Trehalose and Betaine", ANALYTICAL BIOCHEMISTRY, vol. 301, no. 2, 15 février 2002 (2002-02-15), pages 168-174, XP055009246, ISSN: 0003-2697, DOI: 10.1006/abio.2001.5474
- BERNHARD H. WEIGL ET AL: "Non-instrumented nucleic acid amplification assay", PROCEEDINGS OF SPIE, vol. 6886, 1 janvier 2008 (2008-01-01), pages 688604-688604-12, XP055035196, ISSN: 0277-786X, DOI: 10.1117/12.763650
- GE Y ET AL: "Detection of novel swine origin influenza A virus (H1N1) by real-time nucleic acid sequence-based amplification", JOURNAL OF VIROLOGICAL METHODS, ELSEVIER BV, NL, vol. 163, no. 2, 1 février 2010 (2010-02-01), pages 495-497, XP026824839, ISSN: 0166-0934 [extrait le 2009-10-31]
- YOSUKE MIZUNO: "Increased specificity of reverse transcription priming by trehalose and oligo-blockers allows high-efficiency window separation of mRNA display", NUCLEIC ACIDS RESEARCH, vol. 27, no. 5, 1 janvier 1999 (1999-01-01), pages 1345-1349, XP055060414,

## Description

La présente invention concerne un procédé dans lequel au moins un acide nucléique cible, présent dans un échantillon biologique, est amplifié par un procédé d'amplification transcriptionnelle permettant de réunir des étapes de températures différentes, à savoir la dénaturation et l'amplification proprement dite.

L'état de la technique est constitué par un certain nombre d'articles scientifiques qui traite de l'effet thermo-stabilisant des polyols sur les enzymes, c'est notamment le cas de :
- Lee en 1981 dans J. Biol. Chemistry 256(14):7193-7201, intitulé: « The stabilization of proteins by sucrose », qui décrit l'effet thermostabilisant du sucrose sur l'α-chymotrypsin, la chymotrypsinogen et l'ARNase.
- Bernier en 1988 dans J. Biotechnol. 7 :293-298, ayant pour titre : « Stabilization of α-glucosidase by polyhydric alcohols » démontrant l'effet thermo-stabilisant des polyols sur la α-glucosidase.
- Carninci en 1998 dans Proc. Natl Acad. Sci. 95 :520-524, intitulé : « Thermostabilization and thermoactivation of thermolabile enzymes by trehalose and its application for the synthesis of full length cDNA » qui présente la thermo-stabilisation par le tréhalose d'enzymes de transcripton inverse et d'enzymes de restriction.
- Spiess en 2004 dans Clinical chemistry 50 (7) : 1256- 1259, qui titre « Trehalose is a potent PCR enhancer : lowering of DNA melting temperature and thermal stabilization of taq polymerase by disaccharide trehalose », où l'effet thermo-stabilisant du tréhalose sur la Taq polymérase est mis en évidence.

Il est donc clair que depuis une trentaine d'années un nombre important de chercheurs ce sont intéressés à l'effet thermo-stabilisant des polyols sur les enzymes. D'ailleurs une demande de brevet a été déposée voilà quinze ans, sous le numéro EP-A-0.821.058, qui propose une méthode d'amélioration d'une activité enzymatique à température élevée. Le brevet correspondant revendique l'utilisation de polyols pour thermo-stabiliser une polymérase et une enzyme de restriction.

Malgré l'intérêt des scientifiques, il apparaît que personne n'a tenté d'adapter cette approche aux technologies d'amplification transcriptionnelle, du type NASBA, TMA, etc. qui, pour fonctionner, font appel à plusieurs activités enzymatiques différentes. La première étape consiste en la dénaturation de la cible, un acide nucléique, généralement un acide ribonucléique (ARN) à 65°C pendant 2 minutes et la deuxième étape consiste, elle, à ajouter des enzymes nécessaires à l'amplification isothermale à 41°C. Ces deux étapes rendent la méthode techniquement contraignante pour l'utilisateur de part l'utilisation de deux températures successives.

De plus, l'utilisation d'une seule température (41°C) pose actuellement des problèmes divers tels que l'amplification des cibles riches en guanine et cytosine présentant des structure secondaires difficilement amplifiables. La solution la plus connue pour rendre ces structures secondaires facilement amplifiables par les techniques d'amplification transcriptionnelle est l'utilisation d'un cinquième nucléotide dans le mélange d'amplification, qui est la ribo-inosine triphosphate (K Nakahara et al. Nucleic Acids Res. 1998 April 1; 26(7): 1854-1856).

On peut facilement utiliser des cibles d'acide désoxyribonucléiques (ADN) avec la technologie NASBA. Pour ce faire il suffit, par exemple d'appliquer préalablement à l'échantillon traité un procédé selon le brevet EP-B-0.397.269 ou selon la demande de brevet WO-A-02/070735, pour permettre l'amplification des cibles ADN.

Alors que l'homme du métier a utilisé ce type de transcription des cibles d'ADN avec deux étapes de températures différentes pendant des décennies, il ne lui était pas encore venu à l'esprit d'essayer d'améliorer la thermo-stabilité des enzymes utilisées pour diminuer les contraintes de ce type de procédé d'amplification transcriptionnelle.

La présente invention décrit donc une simplification de la méthode d'amplification transcriptionnelle qui est alors réalisée en une seule étape de part l'ajout simultané de l'acide nucléique cible et des réactifs d'amplification, tels que tampons, enzymes, nucléotides, en présence d'additifs chimiques thermo-stabilisants, qui permettent l'utilisation d'une température d'amplification plus élevée avec un alignement avec la température de l'étape de dénaturation desdites cibles. C'est un effet qui est particulièrement inattendu et donc surprenant.

La nouveauté réside en la thermo-stabilisation simultanée de l'ensemble des activités enzymatiques, notamment des trois activités enzymatiques présentes dans la méthode d'amplification NASBA, par l'utilisation d'additifs chimiques de types polyols permettant de préserver les activités de l'ARN polymérase T7, l'ARNase H et l'AMV-RT à des températures plus élevées que 41°C, et permettant ainsi de réunir les étapes expérimentales de dénaturation et d'amplification.

La présente invention propose un procédé d'amplification transcriptionnelle dans lequel :
a) au moins un acide nucléique cible, présent dans un échantillon biologique, est mis en présence :
   - d'amorces d'amplification,
   - de l'ensemble des réactifs nécessaires à la réalisation de l'amplification, dont les enzymes participant à l'amplification, et
   - d'au moins un polyol permettant de stabiliser les enzymes nécessaires à la réalisation de l'amplification,
b) on chauffe le mélange à une température supérieure à 41°C,
c) on réalise une amplification transcriptionnelle de l'acide nucléique cible à une température supérieure à 41°C.

Selon un mode de réalisation du procédé d'amplification, la température à laquelle l'amplification est comprise entre 41 et 49°C.

La présente invention propose un procédé d'amplification transcriptionnelle isotherme tel que défini dans l'une quelconque des revendications 1 ou 2.

Selon un autre mode de réalisation du procédé d'amplification, la température à laquelle l'amplification est réalisée est supérieure ou égale à 46°C.

Quelque soit le mode réalisation du procédé d'amplification, les activités enzymatiques assurées par les enzymes sont :
- l'activité ARN polymérase (T7, SP6, etc.),
- l'activité transcriptase inverse (AMV-RT, MMLV-RT, etc.), et
- l'activité ARNase H.

L'activité ARNase H peut être donnée par une enzyme indépendante (activité dite individuelle) ou par une enzyme ayant une autre activité enzymatique (activité dite associée). Cette autre activité associée à l'ARNase H peut être donnée par une enzyme transcriptase inverse ou ARN polymérase ou une enzyme différente.

Les activités enzymatiques permettent de réaliser des amplifications isothermes, telles que :
- la NASBA (nucleic acid sequence-based amplification),
- la TMA (transcription mediated amplification),
- la 3SR (self-sustained sequence replication),
- la SMART (signal mediated amplification of RNA technology),
- la MDA (Multiple Displacement Amplification), et
- toutes autres amplifications isothermes faisant intervenir au moins une des trois activités citées précédemment et utilisées pour des amplifications du génome entier (whole genome amplification) ou pour des amplifications d'une séquence spécifiques ADN ou ARN).

Quelque soit le mode réalisation du procédé d'amplification, le ou les polyols sont constitués par l'un des composés ou une combinaison des composés suivants :
- lactose,
- sorbitol,
- sucrose,
- mannitol, et
- tréhalose.

Quelque soit le mode réalisation du procédé d'amplification, la concentration en polyol(s) est comprise entre 0,4 et 1,5 M.

La présente invention concerne également un procédé de détection d'amplicons obtenus par le procédé d'amplification, tel que décrit ci-dessus, qui consiste à ajouter durant l'étape a) au moins un type de sonde de détection par acide nucléique cible recherché et susceptible d'être présent dans l'échantillon biologique, et à effectuer l'étape supplémentaire suivante :
d) on effectue la détection de la présence d'amplicons issus de l'amplification réalisée à l'étape c) par hybridation de la sonde sur chaque amplicon en solution.

La présente invention concerne également un procédé de prétraitement du ou des acide(s) nucléique(s) cible(s) recherché(s) et susceptible(s) d'être présent(s) dans l'échantillon biologique, et devant être amplifié(s), tel que décrit ci-dessus, consistant à effectuer l'étape supplémentaire suivante avant l'étape a) dans laquelle ledit l'échantillon biologique est soumis à une température inférieure ou égale à 65°C pour l'ARN et à une température inférieure ou égale à 95°C pour l'ADN.

La présente invention concerne également un procédé de prétraitement du ou des acide(s) nucléique(s) cible(s) recherché(s) et susceptible(s) d'être présent(s) dans l'échantillon biologique, et devant être amplifié(s), tel que décrit ci-dessus, consistant à effectuer une étape supplémentaire, avant l'étape a), dans laquelle ledit l'échantillon biologique est soumis à une température inférieure ou égale à 49°C.

La présente invention concerne également un procédé de diagnostic *in vitro* de la présence d'un ou de différents types d'acide(s) nucléique(s) cible(s) recherché(s) et susceptible(s) d'être présent(s) dans l'échantillon biologique, consistant :
a) à effectuer un procédé de prétraitement, tel que décrit ci-dessus,
b) à effectuer un procédé d'amplification, tel que décrit ci-dessus,
c) à effectuer un procédé de détection, tel que décrit ci-dessus.

Selon un mode de réalisation du procédé de diagnostic, l'ensemble du procédé est réalisé en un seul récipient.

Selon une première variante de réalisation du procédé précédent, l'ensemble du procédé est réalisé à une température unique supérieure à 41°C.

Selon une première variante de réalisation du procédé précédent, l'ensemble du procédé est réalisé à une température unique comprise entre 46 et 49°C.

Les figures ci-jointes sont données à titre d'exemple explicatif et n'ont aucun caractère limitatif. Elles permettront de mieux comprendre l'invention.
Les Figures 1 représentent le criblage fonctionnel de composés thermo-stabilisants lors d'amplifications NASBA à 46°C en présence de 7,5 cp/réaction de transcrits VIH-1B (sept réplicas par criblage), avec dans :
   - la Figure 1A une concentration en lactose de 0,21 M,
   - la Figure 1B une concentration en maltose de 0,9 M,
   - la Figure 1C une concentration en raffinose de 0,05 M,
   - la Figure 1D une concentration en sorbitol de 1,2 M,
   - la Figure 1E une concentration en sucrose 0,6 M, et
   - la Figure 1F une concentration en turanose 1,09 M.
La Figure 2 décrit l'activité résiduelle (%) de l'ARN polymérase T7 après 3 minutes de pré-incubation à différentes température et en présence de différents composes thermo-stabilisants.
La Figure 3A décrit l'activité résiduelle de l'ARN polymérase T7 après 15 minutes de pré-incubation à différentes températures, avec ou sans tréhalose 0,4 M.
La Figure 3B décrit l'activité résiduelle de l'ARNase H après 25 minutes de pré-incubation à différentes températures, avec ou sans tréhalose 0,4 M.
La Figure 3C décrit l'activité résiduelle de l'AMV-RT après 25 minutes de pré-incubation à différentes températures, avec ou sans tréhalose 0,4 M.
La Figure 4 propose la mesure de la sensibilité (%) obtenue en présence de différents composés thermo-stabilisants lors d'une amplification NASBA VIH-1 type B à 5 cps/essai et sans phase de dénaturation de la cible (N=24). A noter qu'à 46°C, le référence ne bénéficiant pas d'additifs thermo-stabilisants ne permet plus d'obtenir d'amplification.

Bien que l'utilisation de sucres et plus généralement des polyols pour thermo-stabiliser les enzymes soit une information qui peut être trouver dans la littérature, leur utilisation à de fortes concentrations dans une amplification transcriptionnelle, telle que la NASBA, pour produire une amplification isotherme à plus de 41°C, notamment à plus de 44°C et préférentiellement à plus de 46°C, avec une pré incubation possible jusqu'à 49°C est une avancée technique permettant de simplifier techniquement ce genre d'amplification pour l'utilisateur final.

Bien que cela fonctionne entre 41 et 45°C, il est notamment nécessaire d'augmenter la température d'amplification NASBA à 46°C afin de faciliter la dénaturation de cibles structurées, permettant ainsi d'améliorer les performances de détection.

Les exemples ci-dessous utilisent la méthode d'amplification transcriptionnelle et isothermale NASBA (Nucleic Acid Sequence Based Amplification). Cependant, l'approche décrite dans ce document est aussi applicable aux autres méthodes d'amplification isothermale telles que la TMA (Transcription Mediated Amplification) ou 3SR (Self-Sustained Sequence Replication) par exemple (Gill et Ghaemi, 2008, Nucleosides, Nucleotides and Nucleic Acids, 27 :224-245 ; Leone et al. 1998, NAR, 26-9 : 2150-2155).

La technologie NASBA est une technologie alternative à la PCR permettant à la différence de cette dernière, la détection génétique des micro-organismes vivants (bactéries, virus, etc...) par amplification d'ARN. Cette technologie d'amplification requiert trois activités enzymatiques pour fonctionner dont l'ARN polymérase T7, l'ARNase H et l'AMV-RT. Parmi ces trois activités enzymatiques, l'ARN polymérase T7 est l'enzyme la plus thermosensible.

### Exemple 1 : Sélection des composés thermo-stabilisants compatibles avec la méthode d'amplification transcriptionnelle et isothermale NASBA

Un ensemble de composés aux propriétés thermo-stabilisantes ou supposées comme telles a été évalué dans un test d'amplification NASBA VIH-1 2.0 sur une plateforme d'amplification Nuclisens EasyQ™ (bioMérieux, Marcy l'Etoile, France) en suivant les recommandations du fournisseurs. 5cp à 30cp d'un transcrit VIH-1 de type B a été utilisé comme cible dans chaque réaction en présence ou absence du composé à évaluer.
L'obtention d'une amplification en présence du composé à une température de 46°C permet de valider le composé comme compatible et thermo-stabilisant pour la réaction NASBA.

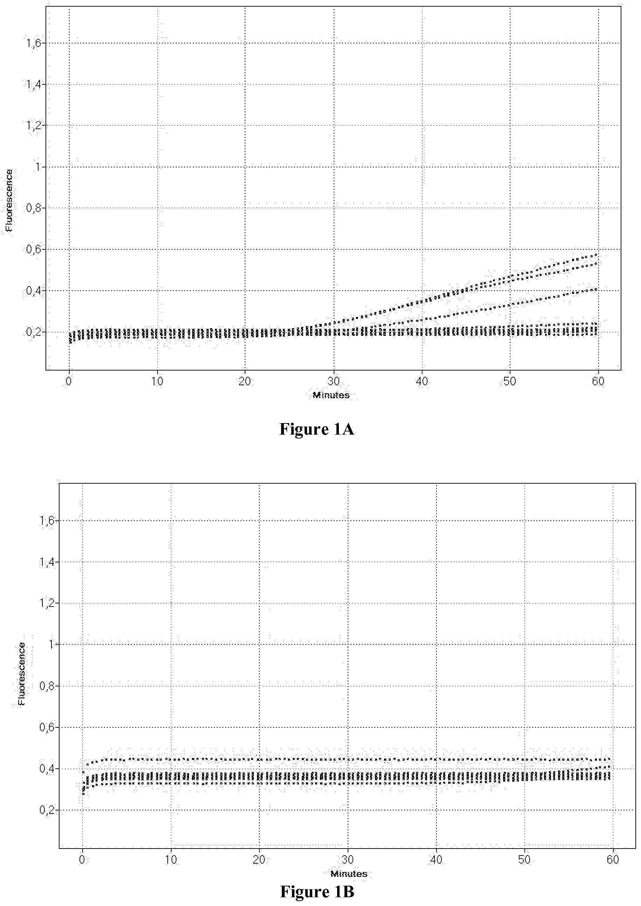

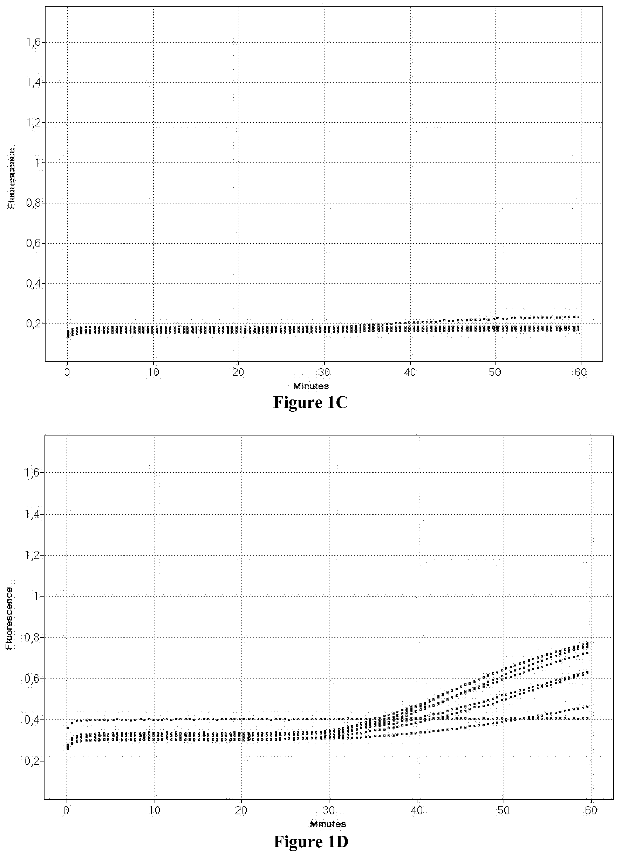

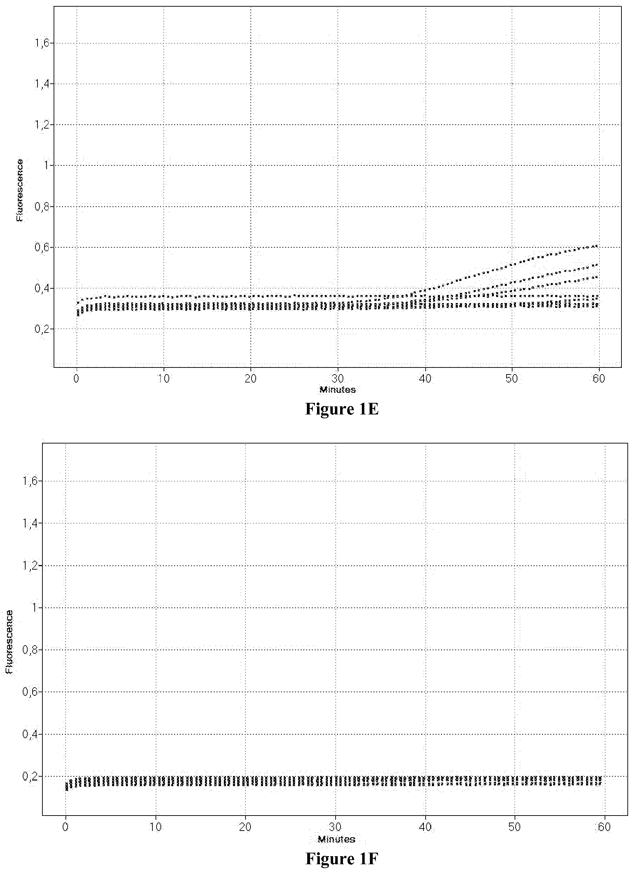

Comme le montrent les exemples des Figures 1A, 1B, 1C, 1D, 1E et 1F, la présence ou l'absence d'une amplification NASBA à 46°C permet de sélectionner aisément les composés thermo-stabilisants tels que le sucrose, sorbitol et lactose qui donnent dans la plupart des cas, une amplification ayant un signal correct (au moins quatre signaux positifs sur sept réplicats). Les additifs ainsi isolés sont étudiés plus précisément par la suite.

### Exemple 2 : Sélection des composés thermo-stabilisants par suivi de la dénaturation thermique de l'ARN polymérase T7 par spectrophotométrie UV

Il est démontré dans cet exemple que la température de dénaturation de l'ARN polymérase T7 (Tm T7) augmente considérablement en présence de certains additifs chimiques, en comparaison de celle du témoin sans additif. L'ARN polymérase T7 a été choisie comme enzyme modèle car elle est la plus sensible à la dénaturation thermique, la Tm T7 sans additif est de 48,5 °C.

Une technique de spectrophotométrie UV est utilisée pour mesurer les Tm T7. L'évolution de l'absorbance de la protéine à λ = 280 nm en fonction de la température est mesurée. Quand l'enzyme est chauffée, la solution devient trouble, des agrégats se forment qui correspondent à la forme dénaturée. La Tm correspond à la température pour laquelle on a 50% de forme native et 50% de forme dénaturée (dérivée première de la courbe absorbance = f(température)).

Dans un flacon en polypropylène, on mélange 4 ml de tampon phosphate PBS 300 mM (Aldrich P-4417, St Quentin Fallavier, France) puis 12 µl d'enzyme ARN polymérase T7 (bioMérieux, Marcy, France) à 17 mg/ml soit une concentration finale en protéine de 0,05 mg/ml. On introduit ensuite dans une cuve en quartz pour spectrophotométrie UV, 500 µl de solution d'ARN polymérase T7 à 0,05 mg/ml et 500 µl d'une solution d'additifs (Aldrich, St Quentin Fallavier, France) concentrée ou de PBS 300 mM pour le témoin. Après homogénéisation, on mesure l'évolution de l'absorbance à λ = 280nm en fonction de la température, entre 30 et 65°C à 1°C/min, afin de déterminer la Tm T7 comme décrit précédemment (Spectrophotomètre UV Cary, Varian, Les Ulis, France).

Quelques résultats représentatifs obtenus d'après la méthode décrite précédemment sont reportés dans le Tableau 1 ci-dessous (les ΔTm sont reportés en fonction du type d'additif).

**Tableau 1 : Tableau récapitulatif des mesures de ΔTm observées en fonction du type d'additif**

| | **Additifs** | **ΔTm (°C)** | **CV (%)** | **Nombre d'expériences** |
|---|---|---|---|---|
| ARN polymérase T7 à 0,05mg/ml | PBS, 150 mM, pH 7,5 | - | 1,6 | 26 |
| idem | PBS, 150 mM, pH 7,5 / Sorbitol 1M | + 0,9 | 1,1 | 3 |
| idem | PBS, 150 mM, pH 7,5 / Sucrose 1M | + 5,8 | 1,0 | 3 |
| idem | PBS, 150 mM, pH 7,5 / Lactose (0,35M) | + 5,5 | 0,2 | 3 |
| idem | PBS, 150 mM, pH 7,5 / Raffinose (0, 085M) | +0,5 | 0,2 | 3 |
| idem | PBS, 150 mM, pH 7,5 / D-Turanose (0,15 M) | -4,4 | 0,1 | 2 |

On observe bien que le chauffage de l'enzyme ARN polymérase T7 en présence de certain additifs comme le sorbitol, le sucrose ou le lactose permet d'augmenter très significativement la température de dénaturation de l'enzyme et cela de plusieurs degrés ce qui confirme sa thermo-stabilisation.

### Exemple 3 : Mesure de thermo-stabilité (T_{1/2}) à 46°C pour l'ARN polymérase T7

Dans cet exemple, les valeurs de T_{1/2} de l'ARN polymérase T7 sont déterminées en présence ou absence de polyols. Une description de la méthode de mesure est présentée ci-dessous, ainsi que les différents réactifs utilisés.

| **Tampon A** | **Tampon B** | **Tampon C** | **Tampon D** |
|---|---|---|---|
| KH₂PO₄/K₂HPO₄ | Tris-HCl 20 mM, pH 7,5 | KH₂PO₄/K₂HPO₄ | Tris-HCl 3,2mM, pH 7,5 |
| 20 mM, pH 7,5 | KCl 300 mM | 200 mM, pH 7,2 | |
| NaCl 100 mM | Tréhalose 1 M | Tréhalose 1 M | NaCl 6,4 mM |
| Tréhalose 1 M | EDTA 7 mM | Triton X-100 0,21 % (w/v) | DTT 0,13 mM |
| EDTA 1 mM | Triton X-100 0,21 % (w/v) | | BSA 1,3 mg/mL |
| Triton X-100 | BSA 0,2 mg/mL | DTT 1 mM | Tréhalose 340 mM |
| 0,268% (v/v) | DTT 1 mM | | |
| BSA 0,1 mg/mL | Acétate de magnésium | | |
| DTT 1mM | 20m M | | |

### Solution W1 :

Mélanger les tampons B, C et D selon les proportions suivantes aux réactifs Nuclisens™ VIH-1 2.0 (réf. : 285033 , bioMérieux, Marcy l'Etoile, France) suivant (pour huit réactions):
- 36,08 µL tampon A
- 6,32 µL tampon B
- 25,36 µL tampon C
- 173,6 µL tampon D
- Huit accusphère « reagent accuspheres Nuclisens™ VIH-1 2.0 »
- 960 µL de diluent « Reagent diluent Nuclisens™ VIH-1 2.0 »

### Solution S (Mix substrat) :

- Tris-HCl à 70 mM, pH 8,5
- dNTP à 1,3 mM chacun
- rATP, rCTP et rUTP à 2,6 mM chacun
- rGTP à 2 mM
- rITP à 0,6 mM
- Saccharose à 60 mM
- Mannitol à 40 mM
- Dextran T-40 à 7 g/L
- MgCl₂ à 16 mM
- KCl à 320 mM
- DTT à 20 mM
- DMSO à 3,5 M
- Balise moléculaire (Molecular Beacon) MB1 entre 0,1 à 0,3 µM
- Oligonucléotide T7-Min entre 10 nM et 20 nM
- Oligonucléotide T7-plus entre 10 nM et 20 nM

Séquences utilisées (orientation 5' - 3'):

| | |
|---|---|
| T7-min | AATTCTAATACGACTCACTATAGTATGAGGGCAGCAGACATCGAATTT |
| T7-plus | AAATTCGATGTCTGCTGCCCTCATACTATAGTGAGTCGTATTAGAATT |
| MB1 | FAM - CTATCCCTTCGATGTCTGCTGCCCTCGGGATAG - Dabcyl |

1. Les enzymes à évaluer sont diluées de telles manières à avoir une activité volumétrique de 109 kU/mL.
2. Un volume de 20 µL de l'enzyme à évaluer sont dilués dans 840 µL de solution W1.
3. Puis 114µL du composé à évaluer sont ajoutés à 193,5 µL de mix enzymatique décrit au point 2 ci-dessus.
4. On fait 12 portions de 20µL du mix qui a été généré au point 3, qui sont réparties dans des tubes de 0,2 mL et incubées à la température de 46°C dans un thermocycleur. Un tube est retiré du thermocycleur toutes les 10 minutes pendant 110 minutes et est stocké à 4°C avant mesure de l'activité enzymatique résiduelle.
5. A 5 µL de ce mélange pré incubé sont additionnés 20 µL de solution S afin de mesurer la vitesse d'augmentation de fluorescence entre 5 et 10 minutes associée à l'activité résiduelle de l'enzyme.
6. L'activité résiduelle de chaque ARN polymérase T7 est exprimée en pourcentage de la fraction d'enzyme n'ayant pas été pré incubé et correspondant au 100% d'activité selon le calcul suivant :
   - ρ_{N} = pente obtenue entre 5 et 10 min pour l'ARN polymérase T7 non pré incubé,
   - ρ_{T} = pente obtenue entre 5 et 10 min pour la ARN polymérase T7 pré incubée à 46°C en présence ou absence de polyols, et
   - % activité relative = % (ρ_{T} / ρ_{N}).
7. La valeur de T_{1/2} correspond au temps qu'il faut pour que l'enzyme ne possède plus que 50% de son activité initiale.

Les résultats de mesure de T_{1/2} de l'ARN polymérase T7 sont décrits dans le Tableau 2 :

**Tableau 2 : Valeurs des thermo-stabilités T_{1/2} de l'ARN polymérase T7 à 46°C**

| | **Molarité finale** | **T_{1/2} 46°C (min)** |
|---|---|---|
| **Référence** | - | 7 |
| **Tréhalose** | 0,40 | 63 |
| **Lactose** | 0,26 | 17 |
| **sucrose** | 0,74 | ∼125 |
| **Sorbitol** | 1,48 | >125 |

D'après le Tableau 2, on constate que tous les composés chimiques évalués ont un effet thermo-stabilisant sur l'activité ARN polymérase T7 fonctionnant dans un environnement propre à la NASBA. Le sorbitol 1,48 M génère l'effet thermo-stabilisant le plus important tandis que le lactose possède un faible pouvoir thermo-stabilisant sur l'ARN polymérase T7, même s'il est visible et significatif.

### Exemple 4 : Mesure de la température maximale de pré-incubation permettant de conserver l'activité ARN polymérase T7

Dans cette exemple, la température maximale que pouvait supporter la ARN polymérase T7 pendant un pré-incubation de 3 minutes en présence de composés thermo-stabilisants est déterminée. Cette étape pouvant être assimilée à une phase de pré-dénaturation ou pré-incubation des cibles.

Le protocole utilisé dans cet exemple est similaire à celui de l'exemple 2 à l'exception du fait que les températures sont variables et que le temps de pré-incubation a été fixé à 3 minutes.

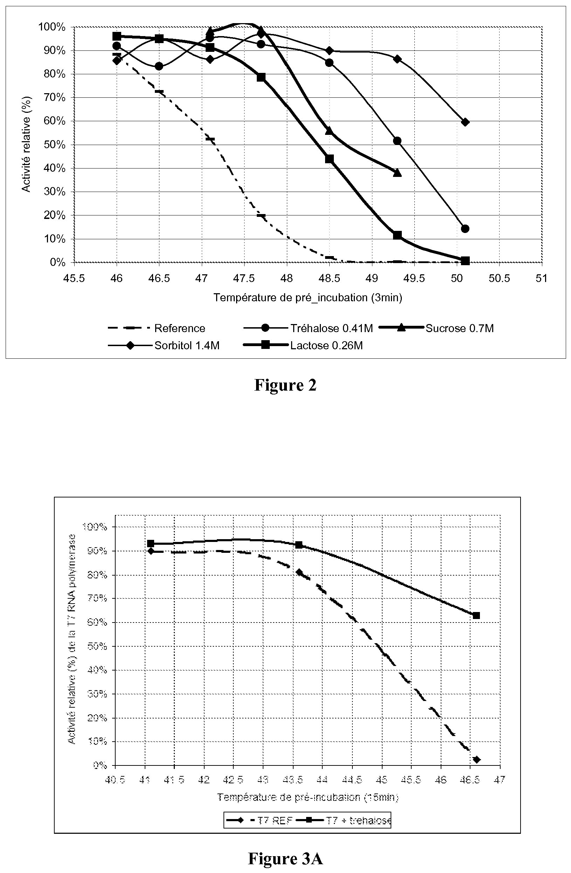

La Figure 2 montre que le sucrose 0,7 M, le tréhalose 0,4 M et le sorbitol 1,4 M permettent de préserver 100% de l'activité ARN polymérase T7 pendant 3 minutes à 48°C, voir 3 minutes à 49°C pour le sorbitol.

### Exemple 5 : Mesure de thermo-stabilité à différentes températures pour l'ARN polymérase T7, l'ARNase H et l'AMV-RT en présence ou absence de tréhalose 0,4 M

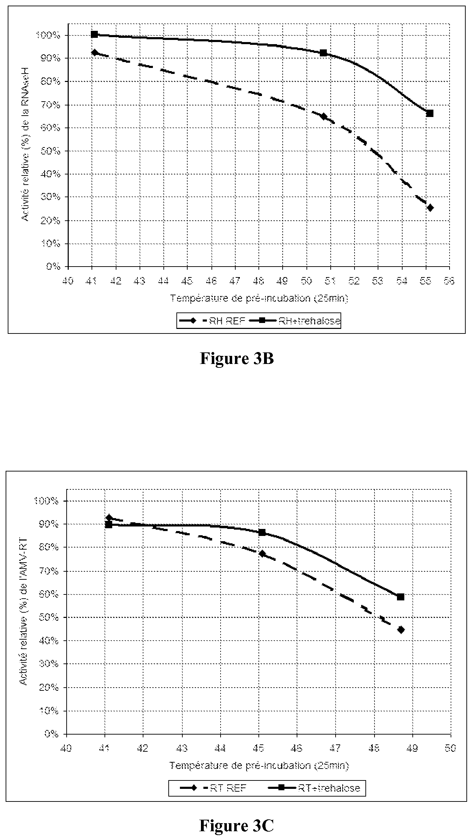

D'une part et comme le montrent les Figures 3A, 3B et 3C, cet exemple met en évidence que l'ARN polymérase T7 (T7) est la plus thermosensible et que l'utilisation d'additif tel que du tréhalose permet de la rendre plus thermostable. D'autre part, on met aussi en évidence l'effet thermo-stabilisant du tréhalose sur les activités ARNase H (RH) et AMV-RT (RT).

Description de la méthode de mesure des activités ARN polymérase T7, ARNase H et AMV-RT pour l'exemple 5:
1. Les enzymes ARN polymérase T7, ARNase H et AMV-RT sont utilisées à des activités volumétriques de 109 kU/mL, 1 kU/mL et 25 kU/mL respectivement.
2. Les enzymes ARN polymérase T7, ARNase H et AMV-RT sont diluées dans la solution W1 de l'exemple 3 selon les ratios 1/43, 2/191 et 2/27 respectivement, afin de mimer l'environnement physicochimique de la réaction NASBA.
3. Aux mélanges précédents sont alors ajouté une solution de tréhalose 1,1 M de manière à avoir une concentration finale de 0,4M.
4. Des portions de 20µL de la solution précédente sont alors réparties dans des tubes de 0.2mL et incubées pendant un temps déterminé à différentes températures.
5. L'activité résiduelle de chacune des activités ARN polymérase T7, ARNase H ou AMV-RT est alors mesurée par addition de 5 µL de solution enzymatique à 20 µL de solution S contenant les réactifs correspondants à chacune des activités mesurées.

### Solution S pour mesurer l'activité ARN Polymerase T7 :

Similaire à exemple 3

### Solution S pour mesurer l'activité ARNase H :

- dNTP à 1,3 mM chacun,
- rATP, rCTP, rUTP à 2,6 mM chacun,
- rGTP à 2 mM,
- rITP à 0,25mM,
- Saccharose à 60 mM,
- Mannitol à 40 mM,
- Dextran T-40 à 7 g/L,
- MgCl2 à 16 mM,
- KCl à 400 mM,
- DTT à 25 mM,
- Tris-HCl à 80mM, pH 8,5,
- DMSO à 4,4M, et
- Sonde RNAseH entre 0,2 et 2 µM: 5' FAM-AUAA-TAMRA 3'.

### Solution S pour mesurer l'activité ADN-dépendante de l'AMV-RT :

- dNTP à 0,3 mM chacun,
- rATP, rCTP, rUTP à 0,6 mM chacun,
- rGTP à 0,5 mM,
- rITP à 0,25 mM,
- Saccharose à 15 mM,
- Mannitol à 10 mM,
- Dextran T-40 à 1,7 g/L,
- MgCl2 à 4 mM,
- KCl à 800 mM,
- DTT à 50 mM,
- Tris-HCl à 165 mM, pH 8,5,
- DMSO à 8,8 M,
- Balise moléculaire MB2 entre 0,2 et 2 µM, et de formule : 5' ROX- GATGCGGAGCGCAGTAGACATGCATCCGAACATCACAG CAGACACAGCCTGGTTTT-DABCYL 3', et
- Oligonucléotide PRT entre 1 et 5 µM et de formule : 5' - AAAACCAGGCTGTGTCTG - 3'

6. L'activité résiduelle de chaque mutant est exprimée en pourcentage de la fraction d'enzyme n'ayant pas été pré incubée et correspondant au 100% d'activité selon le calcul suivant :
- ρ_{N} = pente obtenue entre 5 et 10 min pour l'enzyme non pré incubée,
- ρ_{T} = pente obtenue entre 5 et 10 min pour l'enzyme pré incubée à différentes températures, avec ou sans la présence du polyol à étudier, et
- pourcentage d'activité relative = % (ρ_{T} / ρ_{N}).

### Exemple 6 : Etude de la sensibilité de la détection par méthode d'amplification transcriptionnelle NASBA à 46°C et en présence de composés thermo-stabilisants

Dans cet exemple, il est mis en évidence qu'il est possible de réaliser une amplification NASBA à 46°C en présence de composés thermo-stabilisants et sans phase de dénaturation des transcrits VIH-1B utilisés comme cibles à la concentration de 5 cps/réaction, limite de la détection NASBA. Ceci ayant pour but de simplifier la méthode de part l'ajout simultané des cibles aux mélanges enzymes et réactifs d'amplification.

Un kit d'amplification Nuclisens™ HIV-1 2.0 (Réf. : 285033, bioMérieux, Marcy l'Etoile, France) a été utilisé pour réaliser les expériences d'amplification en présence de tréhalose, sucrose ou sorbitol en limite de détection avec un transcrit VIH-1 type B à 5 cps/essai. Chaque amplification est répliquée 24 fois afin d'estimer la sensibilité de l'essai en présence de composés thermo-stabilisants à 46°C.

La sensibilité est exprimée en pourcentage de signaux positifs déterminés par le système d'analyse EasyQ™ par rapport au nombre total de répliquats.

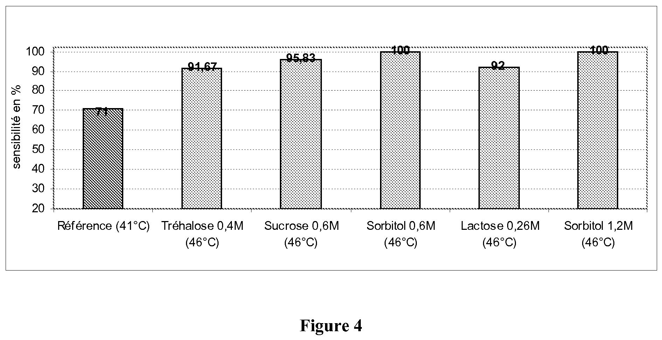

La Figure 4 démontre l'intérêt d'utiliser des températures réactionnelles plus élevées que 41°C en présence de certains polyols de part les gains de sensibilité obtenus, notamment avec le sorbitol qui permet de détecter 100% des transcrits VIH1-B.

## Revendications

1. Procédé d'amplification transcriptionnelle isotherme en une seule étape dans lequel :
a) au moins un acide nucléique cible, présent dans un échantillon biologique, est mis en présence des composés suivants :
• d'amorces d'amplification,
• de l'ensemble des réactifs nécessaires à la réalisation de l'amplification, dont les enzymes participant à l'amplification, et
• d'au moins un polyol permettant de stabiliser les enzymes nécessaires à la réalisation de l'amplification,
b) on réalise une dénaturation des cibles en chauffant le mélange à une température supérieure à 44°C, en présence de l'ensemble des composés.
c) on réalise une amplification transcriptionnelle de l'acide nucléique cible à ladite température supérieure à 44°C,
les étapes de dénaturation et d'amplification étant réunies.

2. Procédé d'amplification, selon la revendication 1, dans lequel la température à laquelle la dénaturation et l'amplification sont réalisées est comprise entre 44 et 49°C.

3. Procédé d'amplification, selon la revendication 1, dans lequel la température à laquelle la dénaturation et l'amplification sont réalisées est supérieure ou égale à 46°C.

4. Procédé d'amplification, selon l'une quelconque des revendications 1 à 3, dans lequel les activités enzymatiques assurées par les enzymes sont :
i. l'activité ARN polymérase (T7, SP6, etc.),
ii. l'activité transcriptase inverse (AMV-RT, MMLV-RT, etc.), et
iii. l'activité ARNase H.

5. Procédé d'amplification, selon l'une quelconque des revendications 1 à 4, dans lequel le ou les polyols sont constitués par l'un des composés ou une combinaison des composés suivants :
i. lactose,
ii. sorbitol,
iii. sucrose,
iv. mannitol, et
v. tréhalose.

6. Procédé d'amplification, selon l'une quelconque des revendications 1 à 5, dans lequel la concentration en polyol(s) est de 0,4 à 1,5 M.

7. Procédé selon la revendication 1 à 5 dans lequel une étape de prétraitement du ou des acides nucléiques cibles recherchés et susceptible(s) d'être présent(s) dans l'échantillon biologique et devant être amplifié(s), est effectuée avant l'étape a) et dans laquelle ledit échantillon biologique est soumis à une température inférieure ou égale à 65°C pour l'ARN et à une température inférieure ou égale à 95°C pour l'ADN.

8. Procédé selon la revendication 1 à 5 dans lequel une étape de prétraitement du ou des acides nucléiques cibles recherchés et susceptible(s) d'être présent(s) dans l'échantillon biologique et devant être amplifié(s), est effectuée avant l'étape a) et dans laquelle ledit échantillon biologique est soumis à une température inférieure ou égale à 49°C.

9. Procédé de détection d'amplicons obtenus par le procédé d'amplification, selon l'une quelconque des revendications 1 à 5, consistant à ajouter durant l'étape a) au moins un type de sonde de détection par acide nucléique cible recherché et susceptible d'être présent dans l'échantillon biologique, et à effectuer l'étape supplémentaire suivante :
d) on effectue la détection de la présence d'amplicons issus de l'amplification réalisée à l'étape c) par hybridation de la sonde sur chaque amplicon en solution.

10. Procédé de diagnostic *in vitro* de la présence d'un ou de différents types d'acides nucléiques cibles recherchés et susceptibles d'être présents dans l'échantillon biologique, consistant :
a) à effectuer un procédé d'amplification, selon l'une quelconque des revendications 1 à 8,
b) à effectuer un procédé de détection, selon la revendication 9.

11. Procédé, selon la revendication 10, **caractérisé par le fait que** l'ensemble du procédé est réalisé en un seul récipient.

12. Procédé, selon l'une quelconque des revendications 10 ou 11, **caractérisé par le fait que** l'ensemble du procédé est réalisé à une température unique supérieure à 44°C.

13. Procédé, selon l'une quelconque des revendications 10 ou 11, **caractérisé par le fait que** l'ensemble du procédé est réalisé à une température unique comprise entre 46 et 49°C.

## Patentansprüche

1. Isothermisches transkriptionsvermitteltes Amplifikationsverfahren in einem einzigen Schritt, wobei:
a) mindestens eine in einer biologischen Probe vorhandene Zielnukleinsäure mit den folgenden Verbindungen zusammengebracht wird:
• Amplifikationsprimern,
• sämtlichen zur Durchführung der Amplifikation benötigten Reagenzien, darunter die an der Amplifikation beteiligten Enzyme, und
• mindestens einem Polyol, mit dem sich die zur Durchführung der Amplifikation benötigten Enzyme stabilisieren lassen,
b) man eine Denaturierung der Ziele durch Erwärmen des Gemischs auf eine Temperatur über 44°C in Gegenwart sämtlicher Verbindungen durchführt,
c) man eine transkriptionsvermittelte Amplifikation der Zielnukleinsäure bei der Temperatur über 44°C durchführt,
wobei die Schritte Denaturierung und Amplifikation zusammengefasst werden.

2. Amplifikationsverfahren nach Anspruch 1, wobei die Temperatur, bei der die Denaturierung und die Amplifikation durchgeführt werden, zwischen 44 und 49°C liegt.

3. Amplifikationsverfahren nach Anspruch 1, wobei die Temperatur, bei der die Denaturierung und die Amplifikation durchgeführt werden, höher als oder gleich 46°C ist.

4. Amplifikationsverfahren nach einem der Ansprüche 1 bis 3, wobei die durch die Enzyme sichergestellten enzymatischen Aktivitäten folgende sind:
i. (T7-, SP6- usw.) RNA-Polymerase-Aktivität,
ii. reverse Transkriptase-Aktivität (AMV-RT, MMLV-RT usw.) und
iii. RNAse H-Aktivität.

5. Amplifikationsverfahren nach einem der Ansprüche 1 bis 4, wobei das Polyol oder die Polyole aus einer der folgenden Verbindungen oder einer Kombination der folgenden Verbindungen besteht/bestehen:
i. Lactose,
ii. Sorbit,
iii. Saccharose,
iv. Mannit und
v. Trehalose.

6. Amplifikationsverfahren nach einem der Ansprüche 1 bis 5, wobei die Konzentration an Polyol(en) 0,4 bis 1,5 M beträgt.

7. Verfahren nach Anspruch 1 bis 5, wobei ein Schritt der Vorbehandlung der Zielnukleinsäure(n), die gesucht wird/werden und von der/denen angenommen wird, dass sie in der biologischen Probe vorhanden ist/sind, und bevor diese amplifiziert ist/sind, vor Schritt a) durchgeführt wird und wobei die biologische Probe einer Temperatur von weniger als oder gleich 65°C für die RNA und einer Temperatur von weniger als oder gleich 95°C für die DNA unterworfen wird.

8. Verfahren nach Anspruch 1 bis 5, wobei ein Schritt der Vorbehandlung der Zielnukleinsäure(n), die gesucht wird/werden und von der/denen angenommen wird, dass sie in der biologischen Probe vorhanden ist/sind, und bevor diese amplifiziert ist/sind, vor Schritt a) durchgeführt wird und wobei die biologische Probe einer Temperatur von weniger als oder gleich 49°C unterworfen wird.

9. Verfahren zum Nachweis von Amplikons, die durch das Amplifikationsverfahren nach einem der Ansprüche 1 bis 5 erhalten werden, bei dem während Schritt a) mindestens ein Typ der Nachweissonde pro Zielnukleinsäure, die gesucht wird und von der angenommen wird, dass sie in der biologischen Probe vorhanden ist, zugegeben wird und der folgende zusätzliche Schritt durchgeführt wird:
d) der Nachweis des Vorhandenseins von Amplikons, die aus der in Schritt c) durchgeführten Amplifikation stammen, durch Hybridisierung der Sonde an jedes Amplikon in Lösung.

10. Verfahren zur *in vitro*-Diagnose des Vorhandenseins eines oder verschiedener Typen von Zielnukleinsäuren, die gesucht werden und von denen angenommen wird, dass sie in der biologischen Probe vorhanden sind, bei dem man:
a) ein Amplifikationsverfahren nach einem der Ansprüche 1 bis 8 durchführt,
b) ein Nachweisverfahren nach Anspruch 9 durchführt.

11. Verfahren nach Anspruch 10, **dadurch gekennzeichnet, dass** das gesamte Verfahren in einem einzigen Gefäß durchgeführt wird.

12. Verfahren nach einem der Ansprüche 10 oder 11, **dadurch gekennzeichnet, dass** das gesamte Verfahren bei einer einzigen Temperatur über 44°C durchgeführt wird.

13. Verfahren nach einem der Ansprüche 10 oder 11, **dadurch gekennzeichnet, dass** das gesamte Verfahren bei einer einzigen Temperatur zwischen 46 und 49°C durchgeführt wird.

## Claims

1. Isothermal transcriptional amplification method carried out in a single step in which:
a) at least one target nucleic acid, present in a biological sample, is placed in the presence of the following compounds:
• amplification primers,
• all the reagents required for carrying out the amplification, including the enzymes participating in the amplification, and
• at least one polyol which makes it possible to stabilize the enzymes required for carrying out the amplification,
b) a denaturation of the targets is carried out by heating the mixture at a temperature above 44°C, in the presence of all the compounds,
c) a transcriptional amplification of the target nucleic acid is carried out at a temperature above 44°C,
the denaturation and amplification steps being combined.

2. Amplification method according to Claim 1, in which the temperature at which the denaturation and the amplification are carried out is between 44 and 49°C.

3. Amplification method according to Claim 1, in which the temperature at which the denaturation and the amplification are carried out is above or equal to 46°C.

4. Amplification method according to any one of Claims 1 to 3, in which the enzymatic activities provided by the enzymes are:
i. the RNA polymerase activity (T7, SP6, etc.),
ii. the reverse transcriptase activity (AMV-RT, MMLV-RT, etc.), and
iii. the RNAse H activity.

5. Amplifikation method according to any one of Claims 1 to 4, in which the polyol(s) consist(s) of one of the compounds or a combination of the compounds which follow:
i. lactose,
ii. sorbitol,
iii. sucrose,
iv. mannitol, and
v. trehalose.

6. Amplification method according to any one of Claims 1 to 5, in which the concentration ofpolyol(s) is from 0.4 to 1.5 M.

7. Method according to any one of Claims 1 to 5 in which a pretreating step of the target nucleic acid(s) which is (are) sought and which may be present in the biological sample, and which must be amplified, is carried out before step a) and in which additional step, said biological sample is subjected to a temperature below or equal to 65°C for RNA and to a temperature below or equal to 95°C for DNA.

8. Method according to any one of Claims 1 to 5 in which a pretreating step of the target nucleic acid(s) which is (are) sought and which may be present in the biological sample, and which must be amplified, is carried out before step a) and in which additional step, said biological sample is subjected to a temperature below or equal to 49°C.

9. Method for detecting amplicons obtained by means of the amplification method according to any one of Claims 1 to 5, which consists in adding, during step a), at least one type of detection probe per target nucleic acid which is sought and which may be present in the biological sample, and in carrying out the following additional step:
a) the detection of the presence of amplicons resulting from the amplification carried out in step c) is carried out by hybridization of the probe on each amplicon in solution.

10. Method for the diagnosis, *in vitro,* of the presence of one type or of various types of target nucleic acids which are sought and which may be present in the biological sample, consisting:
a) in carrying out an amplification method according to any one of Claims 1 to 8,
b) in carrying out a detection method according to Claim 9.

11. Method according to Claim 10, **characterized in that** all of the method is carried out in a single container.

12. Method according to either one of Claims 10 and 11, **characterized in that** all of the method is carried out at a single temperature above 44°C.

13. Method according to either one of Claims 10 and 11, **characterized in that** all of the method is carried out at a single temperature of between 46 and 49°C.
